# EUROPÄISCHE PATENTSCHRIFT

(11) **EP 0 776 888 B1**
(45) Veröffentlichungstag und Bekanntmachung des Hinweises auf die Patenterteilung: **14.05.2003**
(21) Anmeldenummer: 96118312.6
(22) Anmeldetag: 15.11.1996
(51) Int. Cl.: C07D 213/20

(54) **Verfahren zur Herstellung von beta-Hydroxyalkylpicoliniumsalzen sowie Picoliniumsalze als Zwischenprodukte zur Herstellung kationischer Farbstoffe**
Process for the preparation of beta-Hydrosyalkylpicolinium salts and picolinium salts as intermediates for the prepation of cationic dyes
Procédé pour la préparation de sels de beta-Hydroxyalkylpicolinium et sels de picolinium comme intermédiare pour la préparation de colorants cationiques

(30) Priorität: 28.11.1995 DE 19544268
(43) Veröffentlichungstag der Anmeldung: 04.06.1997
(60) Teilanmeldung: 02016939.7
(73) Patentinhaber: BAYER AG, 51368 Leverkusen (DE)
(72) Erfinder: Meisel, Karlheinrich, Dr., 51519 Odenthal (DE); Walz, Klaus, Dr., 51381 Leverkusen (DE); Renner, Gerd-Friedrich, Dr., 51515 Kürten (DE); Schulze, Hans, Dr., 51061 Köln (DE); Gerdes, Carsten, Dr., 51373 Leverkusen (DE); Gassen, Karl-Rudolf, Dr., 40882 Ratingen (DE); Ditzer, Reiner, Dr., 51519 Odenthal (DE); Klein, Lothar, Dr., 51375 Leverkusen (DE)

(56) Entgegenhaltungen:
- EP-A- 0 176 472
- DE-A- 2 230 179
- CHEM.HETEROCYCL.COMP.(ENG.TRANSL.), Bd. 26, Nr. 4, April 1990, Seiten 507-510, XP000652651 GARKUSHA-BOZHKO,V.S. ET AL.: "Reaction of Pyridines with Epichlorohydrin"
- J.AM.CHEM.SOC., Bd. 71, 1949, WASHINGTON, Seiten 3498-3500, XP000652725 KING,L.C.ET AL.: "Reactions of 1,2-Epoxides with Salts of Organic Bases. I. Styren Oxide"
- CHEM.HETEROCYCL.COMP.(ENG.TRANSL.), Bd. 10, Oktober 1984, Seiten 1162-1166, XP000652652 SHISHKIN,G.V.: "Diazabicycloalkanes with nitrogen atoms ..."

## Beschreibung

Die Erfindung betrifft ein Verfahren zur Herstellung, von β-Hydroxyalkylpicoliniumsalzen.

Hydroxyethylpicoliniumsalze sind insbesondere in Form ihrer Chloride, die beispielsweise aus EP-A 176 472 bekannt sind, wichtige Zwischenprodukte bei der Herstellung von kationischen Farbstoffen. Die Chloride werden im allgemeinen durch Umsetzung von Chlorethanol mit Picolinen erhalten. Bei diesem Verfahren wird jedoch das problembehaftete, weil toxische und korrosive, Chlorethanol verwendet.

Aufgabe der vorliegenden Erfindung war es, ein Verfahren zur Herstellung von Hydroxyethylpicoliniumsalzen bereitszustellen, das die genannten Nachteile nicht aufweist.

Es wurde nun ein Verfahren zur Herstellung von β-Hydroxyalkylpicoliniumsalzen der Formel (I) 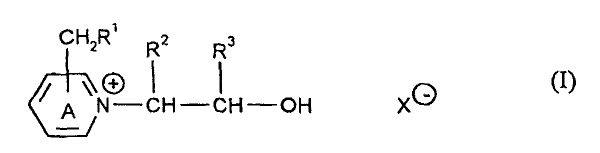 gefunden, wobei der Ring A gegebenenfalls weitere Substituenten trägt und/oder mit carbo- oder heterocyclischen Ringen, vorzugsweise aromatischen, kondensiert sein kann,
- R¹: für H, Cyano, C₁-C₄-Alkoxycarbonyl oder gegebenenfalls substituiertes C₁-C₄-Alkyl, insbesondere C₁-C₂-Alkyl, steht,
- R² und R³: unabhängig voneinander H, oder gegebenenfalls substituiertes C₁-C₄-Alkyl, insbesondere C₁-C₂-Alkyl, bedeuten und
- X^{⊖}: für den Rest eines Äquivalents einer 1- bis 3-basischen Säure steht,
das dadurch gekennzeichnet ist, daß man Picoline der Formel (II) 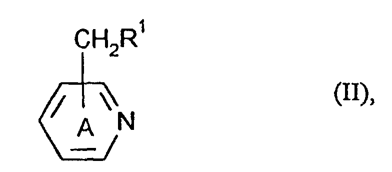 worin R¹ die oben genannte Bedeutung besitzt und der Ring A in oben beschriebener Weise weiter substituiert sein kann, mit Alkylenoxiden der Formel (III) 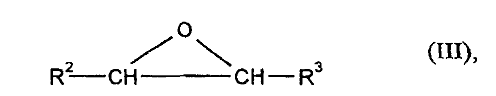 worin R² und R³ die oben genannte Bedeutung besitzen, in Gegenwart einer 1- bis 3-basischen Säure umsetzt, wobei als 1- bis 3-basische Säure H₂SO₄ oder H₃PO₄ eingesetzt wird und dass die Umsetzung in einer CO₂-Atmosphäre durchgeführt wird.

Als geeignete Substituenten für die C₁-C₄-Alkylreste in der Bedeutung von R² und R³ sind beispielsweise CN, Aryloxy wie Phenoxy oder C₁-C₄-Alkoxycarbonyle zu nennen.

In einer besonders bevorzugten Ausführungsform des erfindungsgemäßen Verfahrens werden als Alkylenoxide der Formel (III) Ethylenoxid, Propylenoxid, 1,2-oder 2,3-Butylenoxid eingesetzt.

Ganz besonders bevorzugt ist Ethylenoxid und Propylenoxid, insbesondere Ethylenoxid.

Als bevorzugte Picoline der Formel (II) sind zu nennen: 4-Methylpyridin, 2-Methylpyridin, 4-Methylchinolin, 2-Methylchinolin oder 4-Methyltetrahydrochinolin. Besonders bevorzugt ist 4-Methylpyridin.

Als geeignete weitere Substituenten des Ringes A sind beispielsweise zu nennen: Nitro, Halogen, Cyano, C₁-C₄-Alkyl, C₁-C₄-Alkoxy, Acetylamino oder Dimethylamino. Für den Fall, daß der Ring A mit anderen Ringen kondensiert ist, sind dies vorzugsweise carbocyclische, insbesondere aromatische Benzo- oder Naphthalin-Ringe. Diese sind besonders bevorzugt in 2,3-Stellung des A-Ringes ankondensiert.

Als Reaktionsmedium dient vorzugsweise Wasser oder ein Gemisch aus Wasser und einem polaren organischen Lösungsmittel, wobei das polare organische Lösungsmittel vorzugsweise mit Wasser mischbar, insbesondere in Wasser löslich ist. Als besonders bevorzugte organische Lösungsmittel sind Glykole wie Ethylenglykol, Diethylenglykol, 1,2- und 1,3-Propandiol, Glycerin und Pentaerythrit, Glykolether wie n-Butyl-diethylenglykol, Methoxypropanol, DMF zu nennen. Ganz besonders bevorzugt ist Ethylenglykol oder Propylenglykol (1,2).

Das Mischungsverhältnis von Wasser zu organisch polarem Lösungsmittel kann über große Bereiche variiert werden. Als vorteilhaft hat sich ein Gewichtsverhältnis von 1:3 bis 1:10 erwiesen.

Die Säure kann pur, in konzentrierter oder verdünnter wäßriger Lösung zugegeben werden. Säuregemische sind ebenfalls möglich. Besonders bevorzugt ist H₂SO₄, insbesondere konzentriert (48 bis 98 Gew.-%).

Das Anion X^{e} des Hydroxyethylpicoliniumsalzes leitet sich von der 1- bis 3-basischen Säure ab. So ist beispielsweise auch β-Hydroxyethylsulfat als Anion ein im Rahmen dieser Anmeldung verstandener Abkömmling der 2-basischen Säure H₂SO₄, das gebildet wird, wenn als Alkylenoxid Ethylenoxid verwendet wird.

Das erfindungsgemäße Verfahren wird in der Regel bei einer Temperatur von 0 bis 160°C, insbesondere bei 70 bis 100°C durchgeführt. Das erfindungsgemäße Verfahren kann bei Normaldruck, vermindertem oder erhöhtem Druck durchgeführt werden. Vorteilhaft ist es, die Umsetzung bei Normaldruck oder bei erhöhtem Druck, insbesondere bei 3 bis 6 bar durchzuführen. Eine Druckerhöhung ist insbesondere dann von Vorteil, wenn die Alkylenoxidkomponente der Formel (III) bei der Umsetzungstemperatur gasförmig vorliegt. Dies kann sich insbesondere bei Ethylenoxid oder Propylenoxid vorteilhaft auswirken.

Das erfindungsgemäße Verfahren wird vorzugsweise so durchgeführt, daß das molare Verhältnis der 1- bis 3-basischen Säure zu Alkylenoxid der Formel (II) 1:1 bis 1:2 beträgt. Das molare Verhältnis von Alkylenoxid zu eingesetztem Picolin der Formel (I) beträgt vorzugsweise 1:1 bis 2:1. Selbstverständlich ist die Reaktion auch mit anderen Verhältnissen durchführbar, jedoch führen derartige Verhältnisse zu keinem unerwarteten Vorteil. In einer bevorzugten Verfahrensvariante wird das Picolin der Formel (II) in Wasser oder in einem Gemisch aus Wasser und einem polaren organischen Lösungsmittel als Reaktionsmedium vorgelegt und die Alkylenoxid- und Säurekomponente, vorzugsweise getrennt, zugegeben. In einer bevorzugten Ausführungsform wird so viel Säure unter Bildung des Picoliniumsalzes zugegeben, daß sich ein pH-Wert im Reaktionsmedium von 2 bis 8, vorzugsweise von 4 bis 7 einstellt.

Weiterhin ist es vorteilhaft, die Zugabe von Säure und Alkylenoxid so zu steuern, daß der pH-Wert des Reaktionsgemisches während der Umsetzung in dem genannten pH-Bereich bleibt. Dabei kann die Alkylenoxidzugabe in Abhängigkeit von der Säurezugabe dergestalt erfolgen, daß der pH-Wert im Bereich von 2 bis 8, vorzugsweise von 4 bis 7 gehalten wird.

Besonders bevorzugt erfolgt jedoch die Säurezugabe in Abhängigkeit von der Zugabe des Alkylenoxid (III), wobei die vorteilhaften pH-Bereiche wie oben beschrieben eingehalten werden. Die Ermittlung des pH-Wertes während der Reaktion kann mit pH-Papier oder vorzugsweise mit einer handelsüblichen pH-Elektrode erfolgen. Die Verwendung einer pH-Elektrode hat den Vorteil, daß sie sich in Verbindung mit einer geeigneten Regeleinheit direkt mit einer Zugabe-Einrichtung verwenden läßt, die in Abhängigkeit von dem gewünschten pH-Wert die Zugabe einer der beiden Komponenten wie oben beschrieben steuert. Das erfindungsgemäße Verfahren in einer CO₂-Atmosphäre durchgeführt. Dies führt insbesondere bei Verwendung nichtflüchtiger 1- bis 3-basischer Säuren, wie H₂SO₄, zu Verbindungen der Formel (I), die eine besonders hohe Reinheit besitzen.

Die nach dem erfindungsgemäßen Verfahren erhaltenen Salze können beispielsweise durch destillative Entfernung des verwendeten Reaktionsmediums aus dem Reaktionsgemisch isoliert werden.

### Beispiele

### Beispiel 1

### Apparatur:

Planschliffgefäß mit Bodenablaßventil, Destillationsaufsatz, Anschluß zur Dosierung von CO₂, Ethylenoxid und Schwefelsäure, deren Zugabe über einen Titrator geregelt wird.

### Verfahren:

Unter CO₂-Atmosphäre wurden 480 g 4-Methylpyridin, 316,5 g 1,2-Propandiol und 77,4 g Wasser vorgelegt. Mit konzentrierter Schwefelsäure wurde in dieser Mischung ein pH-Wert von 5 eingestellt. Der Ansatz wurde auf 80°C erwärmt. In dieser Apparatur wurde unter gleichzeitiger Dosierung von Schwefelsäure mit Hilfe eines Titrators bei pH 5 so lange Ethylenoxid eingeleitet, bis ein Schwefelsäureverbrauch von 300 g Schwefelsäure erreicht war. Man ließ den pH-Wert auf 6 ansteigen und leitete nun bei diesem pH-Wert weiter Ethylenoxid ein, bis weitere 50 g Schwefelsäure verbraucht waren. Der Ethylenoxidstrom wurde abgestellt. Durch Reaktion des gelösten Ethylenoxids wurden beim Nachrühren noch 7 g Schwefelsäure verbraucht. Der Gesamtschwefelsäureverbrauch lag bei 357 g. Die Dosierung von Schwefelsäure wurde abgestellt, der Ansatz auf Raumtemperatur abgekühlt und abgelassen. Ausbeute: 1518 g einer Reaktionsmischung folgender Zusammensetzung: Picoliniumsalz 45,6 % (bezogen auf ein Molekulargewicht von 138,2), entspricht 97 % der Theorie, 4-Methylpyridin 1,8 %, Nebenprodukt 1,3 %, Rest Wasser sowie 1,2-Propandiol, β-Hydroxyethylhalogensulfat u.a.

### Beispiel 2

Analog Beispiel 1 wurde das Verfahren durchgeführt, anstatt Schwefelsäure jedoch 540 g konzentrierte Essigsäure eingesetzt. Man erhielt 1810 g eines hellgelben Produktgemisches folgender Zusammensetzung:

| | |
|---|---|
| 1,2 % | 4-Methylpyridin |
| 53,5 % | β-Hydroxyethylpicoliniumacetat |
| 1 % | Nebenprodukt |
| Rest: | Wasser, Propandiol, Ethylenglykolsäure, Essigsäureethylenglykolester |

### Beispiel 3

742 Teile γ-Picolin, 119 Teile Wasser und 489 Teile Propylenglykol werden in einem Rührgefäß vorgelegt und dann unter Kühlung mit 199 Teilen 98%iger Schwefelsäure so versetzt, daß die Temperatur auf 60 bis 70°C ansteigt. Das Reaktionsgefäß wird nun zur Entfernung von Luftsauerstoff mit Stickstoff gespült und auf 80°C erwärmt. Nun werden bei 80 bis 90°C 125 Teile Ethylenoxid innerhalb von ca. 1 Stunde zugegeben. Der pH-Wert der Reaktionsmischung soll dabei nicht über 7 ansteigen. Nach der Zugabe von weiteren 200 Teilen Schwefelsäure werden innerhalb von ca. 1 Stunde nochmals 186 Teile Ethylenoxid zugegeben, bis der pH-Wert der Reaktionsmischung 7,5 bis 8 erreicht hat. Es wird noch 1/2 Stunde nachgerührt und dann unter einem Vakuum von 20 mbar bei 90°C solange destilliert, bis kein Destillat mehr übergeht. Es wird ein flüssiges Produkt erhalten, das neben ca. 30 % Propylenoxid ca. 43 % Hydroxyethylpicoliniumsulfat im Gemisch mit 29 % Hydroxyethylpicolinium-hydroxyethylsulfat enthält. (Gehalt an Hydroxyethylpicoliniumkation: 45 bis 50 %, Bestimmung durch HPLC)

### Beispiel 4

Das Verfahren wurde analog Beispiel 1 durchgeführt, jedoch wurde anstatt Schwefelsäure 357 g Phosphorsäure eingesetzt. Man erhält 1545 g eines Produktgemisches der folgenden Zusammensetzung: Picoliniumsalz: 43,6 %, 4-Methylpyridin: 1,7 %, Nebenprodukt: 1,4 % Rest Wasser, Propandiol, Ethylenglykol und Phosphorsäuremono- und diglycolester.

### Beispiel 5

Aus dem Reaktionsgemisch aus Beispiel 1 werden im Wasserstrahlvakuum bis zu einer Badtemperatur von 100°C

124,4 g einer Mischung aus Wasser und 1,2-Propandiol abdestilliert. Man läßt auf 60°C abkühlen und gibt bei dieser Temperatur 957,7 g 4-Ethylbenzylaminobenzaldehyd und 50 ml Piperidin zu. Anschließend wird auf 80°C erwärmt und bei dieser Temperatur 7 Std. nachgerührt. Am nächsten Morgen wird im Wasserstrahlvakuum 4 Stunden abdestilliert. Man erhält 2467,7 g nicht eingestellten Farbstoffs. Durch Zugabe von 730 g Eisessig und 1459 g Wasser wird die Farbstärke eingestellt.
- Ausbeute:: 4656 g Flüssigeinstellung

## Patentansprüche

1. Verfahren zur Herstellung von β-Hydroxyalkylpicoliniumsalzen der Formel (I) 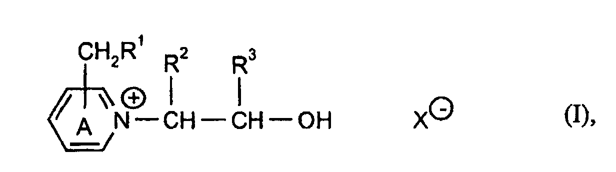 worin
der Ring A gegebenenfalls weitere Substituenten trägt und/oder mit carbooder heterocyclischen Ringen kondensiert ist,
R¹ für H, Cyano, C₁-C₄-Alkoxycarbonyl oder gegebenenfalls substituiertes C₁-C₄-Alkyl steht,
R² und R³ unabhängig voneinander H, oder gegebenenfalls substituiertes C₁-C₄-Alkyl bedeuten und
X^{⊖} für den Rest eines Äquivalents einer 1- bis 3-basischen Säure steht,
**dadurch gekennzeichnet, dass** man Picoline der Formel (II) 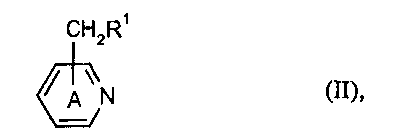 worin A und R¹ die oben genannten Bedeutungen besitzen, mit Alkylenoxiden der Formel (III) 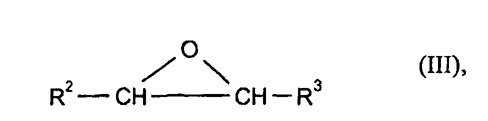 worin R² und R³ die oben genannte Bedeutung besitzen,
in Gegenwart einer 1- bis 3-basischen Säure umsetzt, wobei als 1- bis 3-basische Säure H₂SO₄ oder H₃PO₄ eingesetzt wird, und dass die Umsetzung in einer CO₂-Atmosphäre durchgeführt wird.

2. Verfahren gemäß Anspruch 1, **dadurch gekennzeichnet, dass** man als Alkylenoxide der Formel (III) Ethylenoxid, Propylenoxid, 1,2- oder 2,3-Butylenoxid, einsetzt.

3. Verfahren gemäß Anspruch 1, **dadurch gekennzeichnet, dass** als Picoline der Formel (II) 4-Methylpyridin oder 2-Methylpyridin eingesetzt werden.

4. Verfahren gemäß Anspruch 1, **dadurch gekennzeichnet, dass** die 1- bis 3-basische Säure und das Alkylenoxid der Formel (III) zu den Picolinen gegeben werden.

5. Verfahren gemäß Anspruch 1, **dadurch gekennzeichnet, dass** die Umsetzung bei einem pH-Wert von 2 bis 8 durchgeführt wird.

## Claims

1. Process for the preparation of β-hydroxyalkylpicolinium salts of the formula (I) 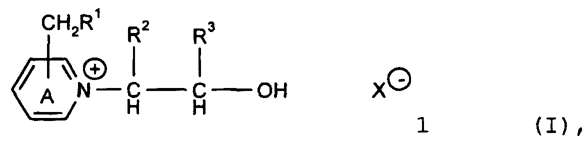 wherein
the ring A optionally carries further substituents and/or is fused with carbo- or heterocyclic rings,
R¹ represents H, cyano, C₁-C₄-alkoxycarbonyl or optionally substituted C₁-C₄-alkyl,
R² and R³ independently of one another denote H, or optionally substituted C₁-C₄-alkyl and
X^{⊖} represents the radical of one equivalent of a 1- to 3-basic acid,
**characterized in that** picolines of the formula (II) 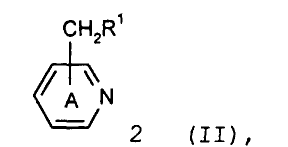 wherein A and R¹ have the abovementioned meanings, are reacted with alkylene oxides of the formula (III) 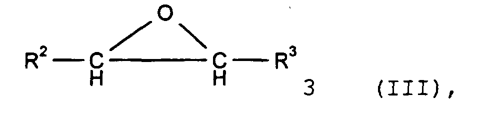 wherein R² and R³ have the abovementioned meaning,
in the presence of a 1- to 3-basic acid H₂SO₄ or H₃PO₄, is employed as the 1- to 3-basic acid and **in that** the reaction is carried out in a CO₂ atmosphere.

2. Process according to Claim 1, **characterized in that** ethylene oxide, propylene oxide, 1,2- or 2,3-butylene oxide are employed as alkylene oxides of the formula (III).

3. Process according to Claim 1, **characterized in that** 4-methylpyridine or 2-methylpyridine are employed as picolines of the formula (II).

4. Process according to Claim 1, **characterized in that** the 1- to 3-basic acid and the alkylene oxide of the formula (III) are added to the picolines.

5. Process according to Claim 1, **characterized in that** the reaction is carried out at a pH of 2 to 8.

## Revendications

1. Procédé pour la préparation de sels de β-hydroxyalkyl-picolinium de formule 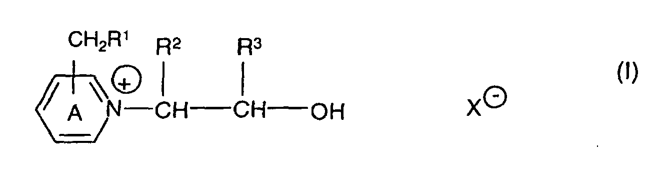 dans laquelle
le cycle A peut le cas échéant porter d'autres substituants et/ou être condensé avec des cycles homogènes ou hétérogènes,
R¹ représente H, un groupe cyano, (alcoxy en C₁-C₄)carbonyle ou un groupe alkyle en C₁-C₄ éventuellement substitué,
R² et R³ représentent chacun, indépendamment l'un de l'autre, H ou un groupe alkyle en C₁-C₄ éventuellement substitué, et
X⊖ représente le radical d'un équivalent d'un acide mono- à tri-basique
**caractérisé en ce que** l'on fait réagir des picolines de formule (II) 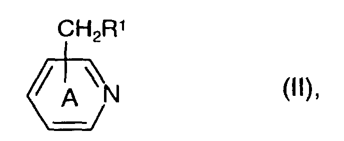 dans laquelle A et R¹ ont les significations indiquées ci-dessus, avec des oxydes d'alkylène de formule (III) 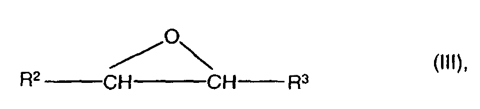 dans laquelle R² et R³ ont les significations indiquées ci-dessus,
en présence d'un acide mono- à tri-basique, l'acide mono- à tri-basique utilisé consistant en H₂SO₄ ou H₃PO₄ et la réaction étant réalisée en atmosphère de CO₂.

2. Procédé selon la revendication 1, **caractérisé en ce que** l'on utilise en tant qu'oxyde d'alkylène de formule (III) l'oxyde d'éthylène, l'oxyde de propylène, l'oxyde de 1,2- ou de 2,3-butylène.

3. Procédé selon la revendication 1, **caractérisé en ce que** la picoline de formule (II) mise en oeuvre est la 4-méthylpyridine ou la 2-méthylpyridine.

4. Procédé selon revendication 1, **caractérisé en ce que** l'on ajoute l'acide mono- à tri-basique et l'oxyde d'alkylène de formule (III) à la picoline.

5. Procédé selon revendication 1, **caractérisé en ce que** la réaction est réalisée à un pH de 2 à 8.
